# EUROPEAN PATENT SPECIFICATION

(11) **EP 4 193 900 B1**
(45) Date of publication and mention of the grant of the patent: **31.07.2024**
(21) Application number: 21212807.8
(22) Date of filing: 07.12.2021
(51) Int. Cl.: A47L 15/00, E05D 15/20

(54) **A MEDICAL WASHER/DISINFECTOR**
MEDIZINISCHES WASCH-/ UND DESINFEKTIONSGERÄT
AGENT DE NETTOYAGE/DÉSINFECTION MÉDICAL

(43) Date of publication of application: 14.06.2023
(73) Proprietor: Getinge Disinfection AB, 351 15 Växjö (SE)
(72) Inventor: JACOBSSON, Ola, 352 61 VÄXJÖ (SE); ENGQVIST, Christoffer, 352 73 VÄXJÖ (SE)
(74) Representative: Zacco Sweden AB

(56) References cited:
- EP-A1- 1 949 843
- EP-A1- 3 075 934
- EP-A2- 2 083 143
- DE-A1- 19 514 303

## Description

### TECHNICAL FIELD

The present invention relates to a medical washer/disinfector comprising a washing chamber for receiving medical articles to be washed/disinfected.

### BACKGROUND ART

Medical washers/disinfectors may typically be provided at dental clinics, surgical departments, central sterile supply departments, and similar facilities. The medical washers/disinfectors may be used for washing/disinfecting a wide range of different medical articles, such as recirculated goods, tubular instruments, surgical sets, anaesthetic items, laboratory items, bedpans, etc. In other words, a washer/disinfector should be able to handle a large variety of different type of goods, which means that sometimes a smaller load size will be processed, and at other occasions a larger load size will be processed. Medical washer/disinfectors are commonly intended to provide both cleaning of visible physical contaminants from medical devices or medical equipment, as well as a degree of disinfection, such as by hot water exposure and/or cleaning/disinfection agents.

A medical washer/disinfector is commonly configured as a cabinet having a washing chamber which is accessible through an opening that may be closed by a door or the like. The opening is normally located at a distance from the ground, such as extending from approximately the height of the waist of a person and upwards. This is a convenient working height for the staff handling the medical washer/disinfector. Articles may, for instance, be brought to the medical washer/disinfector by means of a cart from which they are unloaded and loaded into the medical washer/disinfector. Another loading possibility is using a conveyor belt or the like which transports the articles into the medical washer/disinfector.

As explained above, medical washers/disinfector may be provided at various different facilities. Sometimes the available space for installing a medical washer/disinfector is rather limited. Such limited available space puts a limitation on the dimension of the medical washer/disinfector. Not only is the dimension relevant to consider in a closed state, but also in an open state, since the available displacement of the door from its closed position to its open position may also be restricted by the facility at which the medical washer/disinfector is to be installed. It would therefore be desirable to provide a medical/disinfector which takes such limited space facilities into consideration.

### SUMMARY OF THE INVENTION

An object of the present invention is to provide a medical washer/disinfector which at least partly alleviates the drawbacks of the prior art and which enables installation in facilities having limited available space. This and other objects, which will become apparent in the following, are accomplished by a medical washer/disinfector as defined in claim 1. Exemplary embodiments are presented in the dependent claims.

The general inventive concept is based on the realization that the distance below the level of the opening to the washing chamber may be utilized for displacement of the door to its open position. By making use of this space below the opening, no substantial additional space is required for opening the door, as opposed to swinging the door open by a pivoting motion, or raising the door above the top of the medical washer/disinfector which needs a large distance to the ceiling of the facility. Furthermore, it has been realized that since a vertical movement of the door should suitably be performed with a small play between the door and the material surrounding the opening (to avoid friction/chafing) an upwardly directed force provided by a motor to raise the door may also be used for closing such play. In particular, the inventor has realized that the upwards force caused by the motor may be used to raise the door and at the appropriate raised position the continued upward force is transferred into a horizontal closing force. Thus, even though a continued upwards force is provided by the motor, the door will not be raised too high. The general inventive concept and exemplary embodiments thereof will now be discussed in more detail.

According to a first aspect of the present invention as in claim 1, there is provided a medical washer/disinfector, comprising:
- a washing chamber for receiving medical articles to be washed/disinfected,
- an opening for accessing the interior of the washing chamber,
- a door which is movable
   ∘ from a lowered open position, enabling medical articles to be inserted into or retrieved from the washing chamber through the opening,
   ∘ and then to a raised stop position, in which the door is located in front of the opening but positioned laterally spaced apart from the opening,
   ∘ and then to a raised closed position, in which the door is positioned against the opening and seals the opening,
   wherein the door also passes through the raised stop position when transitioning from the raised closed position back to the lowered open position, the door comprising of being fixedly connected to a first slide surface,
- a motor,
- a carrier arrangement vertically movable by the motor, the carrier arrangement comprising a second slide surface for enabling slidable contact with the first slide surface,
- a spring member connected to the door at a connection area, the spring member being in contact with the carrier arrangement and extending vertically upwardly from the carrier arrangement to said connection area,

wherein when the door is in the lowered open position and the motor moves the carrier arrangement upwards, the door is pushed upwards by the spring member until the door reaches said raised stop position at which point further upwards movement of the door is prevented, wherein the pushing upwards by the spring member prevents sliding motion between the first and second slide surfaces during the movement of the door from the lowered open position to the raised stop position,
wherein, after the door has reached the raised stop position, continued upward movement of the carrier arrangement causes the spring member to become more compressed, thereby enabling the first slide surface to slide along the second slide surface, whereby the door moves horizontally from the raised stop position to the raised closed position due to the continued upward movement of the carrier arrangement.

By opening the door downwardly to a lowered position, and raising the door upwardly and allowing the door to be subject to the upwardly directed force stemming from the motor but transferring it to a horizontal closing motion, a compact yet efficient medical washer/disinfector is obtainable. The spring member will be in a substantial steady state during the upwards motion of the door pushing the door upwardly and keeping it at a substantially fixed lateral distance from the opening. Thus, the spring member keeps the door from self-locking during the movement from the lowered open position to the raised stop position (and also during the reverse movement, i.e. from the raised stop position to the lowered open position). However, when the door is prevented from moving further upwardly, the continued upwards movement of the carrier arrangement results in that the carrier arrangement approaches the connection area of the door. Due to this closing-in of the carrier arrangement, the spring member (extending between the carrier arrangement and the connection area door) becomes more compressed, and cannot keep the door laterally spaced from the opening. Instead the door will now by means of the first and second slide surfaces move horizontally to close the opening.

According to at least one exemplary embodiment, at least one of the first and second slide surfaces is inclined such that it is at a higher vertical level distally to the washing chamber and at a lower vertical level proximally to the washing chamber. By having at least one slide surface inclined an efficient horizontal motion will be achieved as the spring becomes gradually more and more compressed as the motor continues driving the carrier arrangement upwardly.

Suitably both the first and second slide surfaces are inclined, and suitably at the same angle of inclination, thereby providing for a larger interface between the slide surfaces. The inclination may suitably be straight, such as forming an angle of inclination of 30°-60° relative to a vertical extension. For instance, for an angle of inclination of 45°, when the door has reached the raised stop position, the door would move one unit length (e.g. 1 mm) in the horizontal direction for each unit length (e.g. 1 mm) that the carrier arrangement continues to move upwards in the vertical direction. It should be understood, that other inclinations of the first and/or second slide surfaces are conceivable, such as for instance, a curved inclination.

According to at least one exemplary embodiment, the medical washer/disinfector comprises at least one slide member fixedly connected to a lateral side of the door, wherein the slide member comprises said first slide surface, wherein said first slide surface is inclined such that it is at a higher vertical level distally to the washing chamber and at a lower vertical level proximally to the washing chamber. By having a slide member fixed to the door is convenient from a manufacturing perspective, as it may be easier to provide said first slide surface on a separate member than on the door itself. The inclination of the first slide surface on the slide member may have anyone of the previously discussed configurations, or in any other appropriate configuration.

According to at least one exemplary embodiment, the carrier arrangement comprises a first carrier element and a second carrier element, the first carrier element being located above and vertically spaced apart from the second carrier element, wherein the spring member is in contact with the first carrier element and extending vertically upwardly from the first carrier element, wherein an upper side of the second carrier element presents said second slide surface. By separating the carrier arrangement into at least a first and a second carrier element, the different functionalities may be better taken into account. The second slide surface on the second carrier element may be designed independently of the surface on the first carrier element which may be design for appropriately supporting the spring member. For exemplary embodiments that include the previously mentioned slide member that is fixedly connected to the door, such a slide member may suitably be arranged between the first and second carrier elements.

According to at least one exemplary embodiment, the medical washer/disinfector comprises a gasket around the opening, wherein the door seals against the gasket when it is in its raised closed position. The gasket provides an improved sealing interface against the door.

According to at least one exemplary embodiment, said spring member is a first spring member, the medical washer/disinfector further comprising a second spring member, wherein the second spring member is configured to accumulate elastic potential energy when the door is moved from the raised stop position to the raised closed position, wherein when the door is in the raised closed position and the motor starts to move the carrier arrangement downwardly, the second spring member releases the elastic potential energy to separate the door from the gasket. This is advantageous since the gasket may provide some degree of "stickiness", which may result in that the door does not separate well enough from the gasket when moving from the raised closed position to the raised stop position and then to its lowered open position. The second spring member will thus give the door an extra push to make sure there is a play between the gasket and the door during the vertical downwards movement of the door.

According to at least one exemplary embodiment, said second spring member comprises a turnable wheel and a spring portion, wherein the turnable wheel is connected to the carrier arrangement and wherein the spring portion interconnects the turnable wheel with the door, wherein when the door is moved from the raised stop position to the raised closed position, the spring portion pulls and turns the turnable wheel. This provides an efficient mechanism for ensuring the above mentioned separation of the door from the gasket.

According to at least one exemplary embodiment, the motor comprises a self-locking gearbox, thereby preventing the carrier arrangement from moving uncontrollably downwards when the door is in the raised closed position. Thus, the risk of the door not sealing properly during a washing/disinfecting process is avoided. It should, however, be understood that there are other conceivable alternatives for preventing such uncontrollable downwards motion. For instance, separate locking elements may be provided for engaging with the carrier arrangement.

As has already been understood from the above discussion, in order to avoid unnecessary friction during the vertical movement of the door, there should suitably be a play between the door and the area around the opening during such vertical movement. This is at least partly reflected in the following exemplary embodiment, according to which, when the door is moved from the lowered open position to the raised stop position, the door substantially floats on the spring member so that the second slide surface substantially does not press against or slide along the first slide surface.

Similarly, as has already been understood from the above discussion, when the door is moved upwardly and reaches the raised stop position, the continued upward vertical movement of the carrier arrangement will instead be transferred into a horizontal movement of the door. This is at least partly reflected in the following exemplary embodiment, according to which when the door is moved from the raised stop position to the raised closed position, vertical movement of the door is substantially prohibited, the spring member is compressed, and upwards pushing of the second slide surface against the first slide surface causes sliding by the first slide surface which moves the door horizontally into the raised closed position.

According to at least one exemplary embodiment, the medical washer/disinfector further comprises:
- a limit switch, and
- a shuttle configured to move in the opposite vertical direction relative to the carrier arrangement, and configured to engage and activate the limit switch during its downwards vertical movement when the door has moved from the raised stop position to the raised closed position,

wherein activation of the limit switch stops the carrier arrangement from moving further vertically upwards and thus stops the door from moving further towards, or pressing further against, the area around the opening,
wherein the vertical position of the limit switch is adjustable, thereby enabling adjustment of the sealing pressure of the door.

This is advantageous as it may be desirable to have different sealing pressures for different applications.

As can be understood from the previous discussion, the force provided by one motor is enough to achieve both the vertical movement of the door from the lowered open position to the raised stop position and the horizontal movement of the door from the raised stop position to the raised closed position. Thus, according to at least one exemplary embodiment, the medical washer/disinfector comprises only one motor for moving the door.

The motor disclosed herein may suitably be controlled by a control unit. The control unit may receive input commands from a user interface, and control the motor based on such input commands. The user interface may, for instance, be a control panel on the medical washer/disinfector and/or a remotely located input device (such as a remote computer or cell phone).

The control unit may include a microprocessor, microcontroller, programmable digital signal processor or another programmable device. The control unit may also, or instead, include an application specific integrated circuit, a programmable gate array or programmable array logic, a programmable logic device, or a digital signal processor. Where it includes a programmable device such as the microprocessor, microcontroller or programmable digital signal processor mentioned above, the processor may further include computer executable code that controls operation of the programmable device. The control unit is preferably configured for use with medical washer/disinfectors, and provided with electronic instructions to perform and control the opening and closing of the door.

### BRIEF DESCRIPTION OF THE DRAWINGS

Fig. 1 illustrates a medical washer/disinfector in an open state, in accordance with at least one exemplary embodiment of the invention.
Fig. 2 illustrates the medical washer/disinfector of Fig. 1 in a closed state.
Fig. 3 illustrate certain components involved in effecting a change from the open state to the closed state of the medical washer/disinfector, in accordance with at least one exemplary embodiment of the invention.
Fig. 4 is a zoomed-in view of Fig. 3, showing the door in its raised stop position.
Fig. 5 corresponds to the view in Fig. 4 but showing the door in it its raised closed position.
Fig. 6 illustrates a detailed view of a mechanism for facilitating the door to return from its raised closed position to its raised stop position.
Fig. 7 illustrates a detailed view of a mechanism enabling adjustment of the sealing pressure.
Fig. 8 illustrates a detailed view of the location of the motor.

### DETAILED DESCRIPTION

The present inventive concept will now be described more fully hereinafter with reference to the accompanying drawings, in which certain aspects of the inventive concept are shown. The inventive concept may, however, be embodied in many different forms and should not be construed as limited to the embodiments and aspects set forth herein; rather, the embodiments are provided by way of example so that this disclosure will be thorough and complete, and will fully convey the scope of the inventive concept to those skilled in the art. Accordingly, it is to be understood that the present inventive concept is not limited to the embodiments described herein and illustrated in the drawings; rather, the skilled person will recognize that many changes and modifications may be made within the scope of the appended claims. Like reference numerals refer to like elements throughout the description.

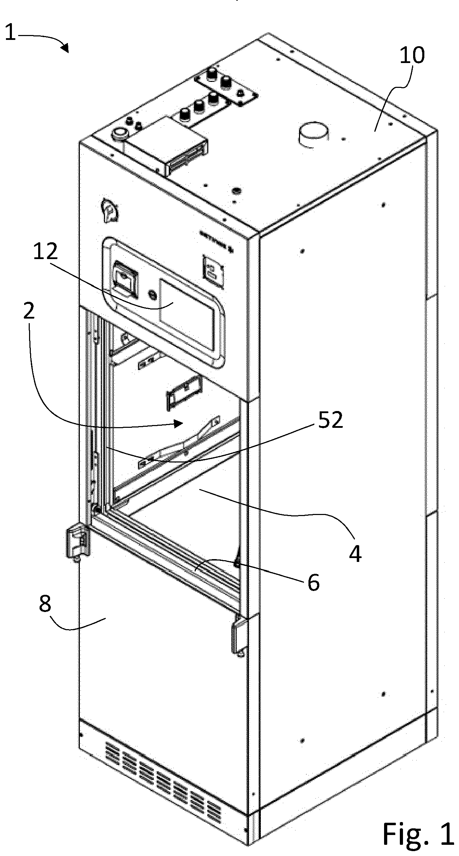

Fig. 1 illustrates a medical washer/disinfector 1 in an open state, in accordance with at least one exemplary embodiment of the invention. The medical washer/disinfector 1 comprises a washing chamber 2 for receiving medical articles to be washed/disinfected. An opening 4 is provided for accessing the interior of the washing chamber 2. The medical washer/disinfector 1 will normally be standing on a floor in a facility, and the opening 4 is located at an elevated level from the floor to allow for a convenient working height for a user. The medical washer/disinfector 1 is also provided with a movable door 6. In Fig. 1 only a top surface of the door 6 is discernible. The rest of the door 6 is located behind a front panel 8 of the medical washer/disinfector 1. Thus, in this illustrated open state of the medical washer/disinfector 1, the door 6 is in a lowered open position, enabling medical articles to be inserted into or retrieved from the washing chamber 2 through the opening 4. This is advantageous as the vertical space below the opening 4 is needed anyway for providing a convenient working height, and the inventive concept makes use of this available vertical space by allowing the door 6 to move downwardly for giving an operator access to the washing chamber 2. If the door 6 would instead have opened upwardly, it would need to extend past the top 10 of the medical washer/disinfector 1, which may not be practically possible when installed in facilities having a low ceiling.

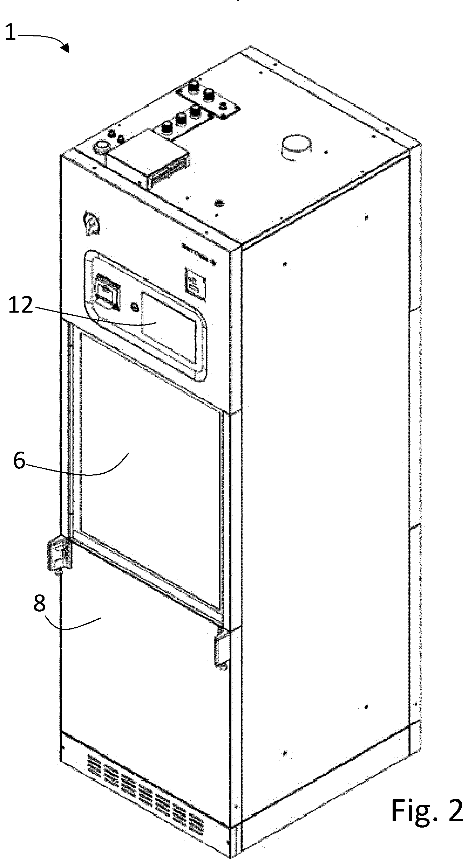

The door 6 is movable from the lowered open position to a raised stop position in which the door 6 is located in front of the opening 4 but positioned laterally spaced apart from the opening, and then to a raised closed position in which the door 6 is positioned against the opening 4 and seals the opening 4. This raised closed position is illustrated in Fig. 2. After a completed washing/disinfection program, the door 6 is moved in the reverse order. Thus, the door 6 will move through the raised stop position when transitioning from the raised closed position (Fig. 2) back to the lowered open position (Fig. 1).

The displacement of the door 6 may be activated by an operator, either directly or indirectly (for example by selecting a washing/disinfecting program). The operator may provide instructions to the medical washer/disinfector 1 via a user interface 12. In Figs. 1 and 2, a user interface 12 is exemplified in the form of a control panel on the medical washer/disinfector 1, however, other local or remote user interfaces are conceivable alternatives or supplements.

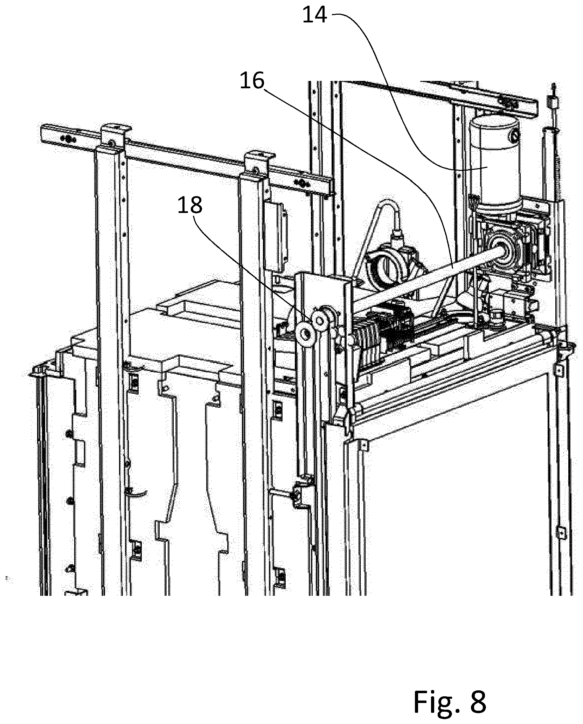

The displacement of the door 6 is powered by a motor. An example of such a motor 14 is illustrated in Fig. 8. The rotary motion of the motor 14 may be transmitted/translated to a rotary motion of a shaft 16 connected to one or more with pulleys 18 for moving a band, belt, chain, or the like to which a carrier arrangement is connected. The motor 14 may suitably comprise a self-locking gearbox, thereby preventing the carrier arrangement from moving uncontrollably downwards when the door 6 is in the raised closed position.

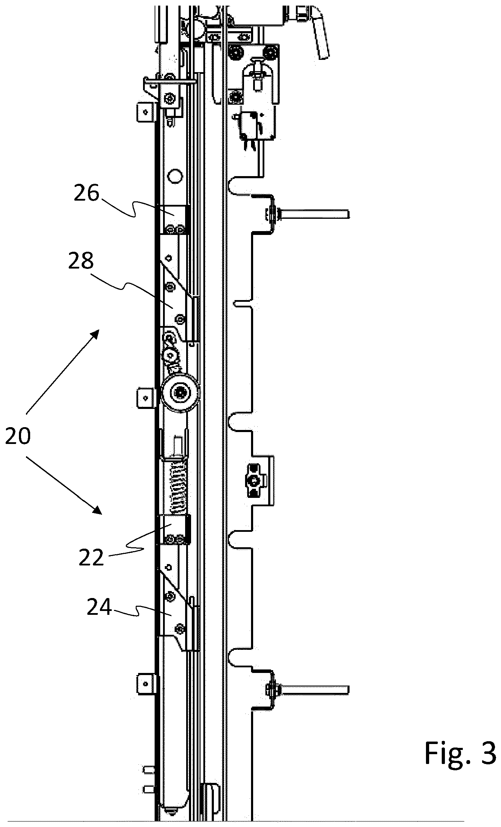

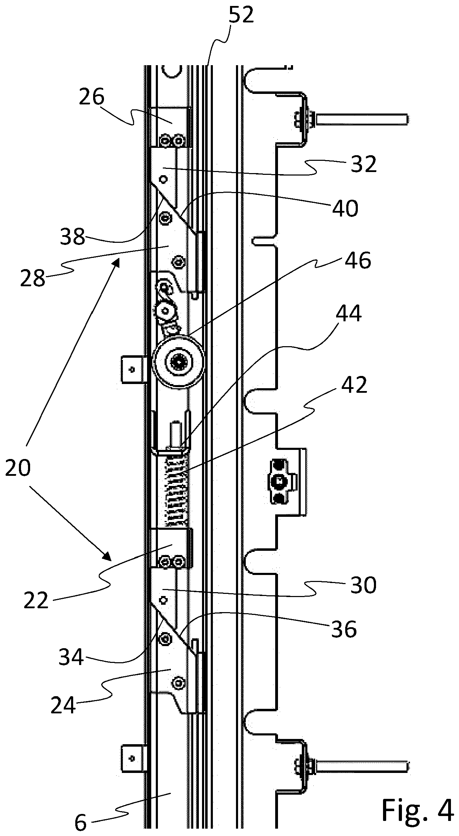

Fig. 3 illustrates an example of such a carrier arrangement 20. The view of Fig. 3 is a detailed side view from the right side of Fig 1, as seen from within the outer visible housing of Fig. 1. The carrier arrangement 20 is vertically movable by the motor 14 (via the shaft 16, pulleys 18, band, etc.), though not illustrated in Figs. 3. In the illustrated exemplary embodiment the carrier arrangement 20 comprises a first carrier element 22 and a second carrier element 24, the first carrier element 22 being located above and vertically spaced apart from the second carrier element 24. The carrier arrangement 20 may further comprise a third carrier element 26 and a fourth carrier element 28, the third carrier element 26 being located above and vertically spaced apart from the fourth carrier element 28. The fourth carrier element 28 is in its turn located above and vertically spaced apart from the first carrier element 22. The carrier arrangement 20 with its first, second, third and fourth carrier elements 22, 24, 26 and 28, respectively, can be seen more clearly in the zoomed-in view of Fig. 4.

The door 6 may comprise a first slide surface, or as illustrated in Fig. 4, the door 6 may comprise a slide member 30 fixedly connected to a lateral side of the door 6 (i.e. not the front, back, top or bottom sides of the door) and that slide member 30 may comprise such a first slide surface 34. The slide member 30 is a lower slide member 30, as there is also provided an upper slide member 32. The lower slide member 30 is located between the first carrier element 22 and the second carrier element 24. The upper slide member 32 is located between the third carrier element 26 and the fourth carrier element 28. Suitably, the opposite lateral side of the door 6 has a corresponding configuration with slide members and carrier elements.

Nevertheless, it should be understood that the number of carrier elements may be different than what has now been illustrated. For instance, in some exemplary embodiments the third and fourth carrier elements 26, 28, as well as the upper slide member 32 may be omitted, while in other exemplary embodiments, there may be provided additional carrier elements and slide member(s).

According to the general inventive concept, the carrier arrangement 20 comprises a second slide surface for enabling slidable contact with the first slide surface 34. In this exemplary embodiment, the second carrier element 24 presents such a second slide surface 36 forming an interface with the first slide surface 34 of the lower slide member 30. In particular, it is the upper side of the second carrier element 24 that presents said second slide surface 36 which mates with the first slide surface 34, which is located at a lower side of said lower slide member 30. According to at least some exemplary embodiments, such as the one illustrated in Fig. 4 (but also in others), the second slide surface 36 has a longer extension than the first slide surface 34. In particular, the second slide surface 36 may have a longer extension than the first slide surface 34 as seen in the sliding direction.

Similarly to the second carrier element 24, the fourth carrier element 28 has a corresponding second slide surface 40 mating with the first slide surface 38 of the upper slide member 32.

As can be seen in Fig. 4, each one of the first and second slide surfaces 34, 36, 38, 40 is inclined such that it is at a higher vertical level distally to the washing chamber and at a lower vertical level proximally to the washing chamber. This enables a good sliding of the first slide surfaces 34, 38 along the respective second slide surfaces 36, 40. It should, however, be understood, that a similar sliding motion (directionwise) could also be achieved, if only one of the mating first and second slide surfaces 34, 36, 38, 40 (in each pair) would have such an inclination.

The medical washer/disinfector 1 also comprises a spring member, which in this exemplary embodiment is illustrated as a first spring member 42 (see Fig. 4), which is connected to the door 6 at a connection area 44. The first spring member 42 is in contact with the carrier arrangement 20. In this particular exemplary embodiment, the first spring member 42 is in contact with the first carrier element 22 of the carrier arrangement 20. The first spring member 42 extends vertically upwardly from the first carrier element 22 to said connection area 44.

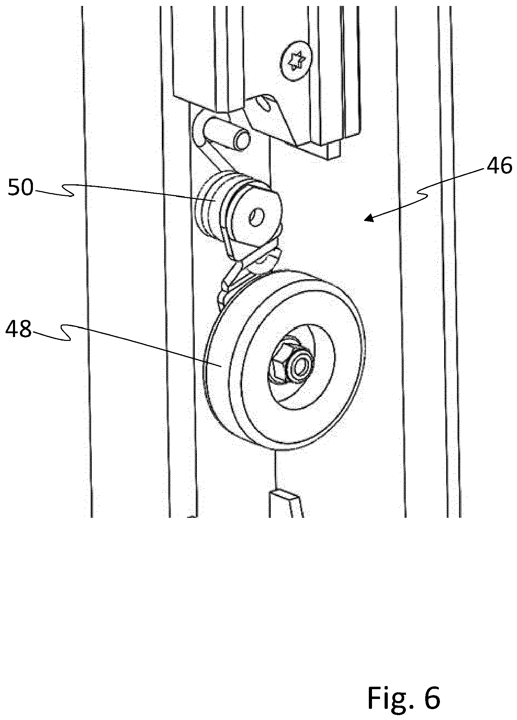

The medical washer/disinfector 1 also comprises a second spring member 46 (see for example Fig. 4 and 6). As best seen in Fig. 6, the spring member 46 comprises a turnable wheel 48 and a spring portion 50. The turnable wheel 48 is connected to the carrier arrangement 20 and the spring portion 50 interconnects the turnable wheel 48 with the door 6. Although not seen in the drawings, the carrier arrangement 20 may suitably comprise a beam connected to the carrier elements, suitably extending all the way from the second carrier element 24 to the third carrier element 26 (and being located closer to the viewer of the drawing figure than the plane currently viewed in Fig. 4). Such a beam may, for instance, be fastened to one or more of the carrier elements 22, 24, 26, 28 by means of the illustrated bolts shown in the carrier elements 22, 24, 26, 28. Such a beam will thus follow the motions of the carrier elements 22, 24, 26, 28. The turnable wheel 48 of the second spring member 46 may suitably be connected to such a beam of the carrier arrangement 20. In the presently illustrated exemplary embodiment, the second spring member 46 is located between first spring member 42 and the fourth carrier element 28. It should, however, be understood that other locations of the second spring member 46 are also conceivable while still performing its intended function, which will be explained further below in this disclosure.

In the illustrated exemplary embodiment, the medical washer/disinfector 1 further comprises a gasket 52 (see e.g. Figs. 1 and 4) around the opening 4, wherein the door 6 is configured to seal against the gasket 52 when the door 6 is in its raised closed position.

In operation, when the door 6 is in the lowered open position (Fig. 1) and the motor 14 (Fig. 8) moves the carrier arrangement 20 (Fig. 4) upwards, the door 6 is pushed upwards by the first spring member 42. Despite the inclined interface between the mating first slide surfaces 34, 38 (facing downwardly) and the respective second slide surface 36, 40 (facing upwardly), the door 6 remains substantially stationary in the horizontal direction during its upward motion, i.e. the horizontal separation between the door 6 and the opening 4 remains substantially the same during the upwards motion of the door 6. This is due to the first spring member 42. The first spring member 42 prevents sliding motion between the first and second slide surfaces 34, 36, 38, 40 during the movement of the door 6 from the lowered open position to the raised stop position illustrated in Fig. 4. Thus, the first spring member 42 is strong enough to withstand the gravitational effect on the door 6. Without the first spring member 42 the door 6 would become self-closing, as the slide members 30, 32 on the door 6 would, due to gravitation, slide along the respective carrier elements 36, 40. Accordingly, during the movement of the door 6 from the lowered open position to the raised stop position of Fig. 4, the slide members 30, 32 will be located at the relatively distal position shown in Fig. 4, i.e. distal relative to the opening 4 and the surrounding gasket 52.

From the above it can be understood that the door 6 can be regarded as substantially floating on the first spring member 42. This floating will continue throughout the upwards motion of the door 6. The door 6 is pushed upwards by the first spring member 42 until the door 6 reaches the raised stop position (shown in Fig. 4) at which point further upwards movement of the door 6 is prevented.

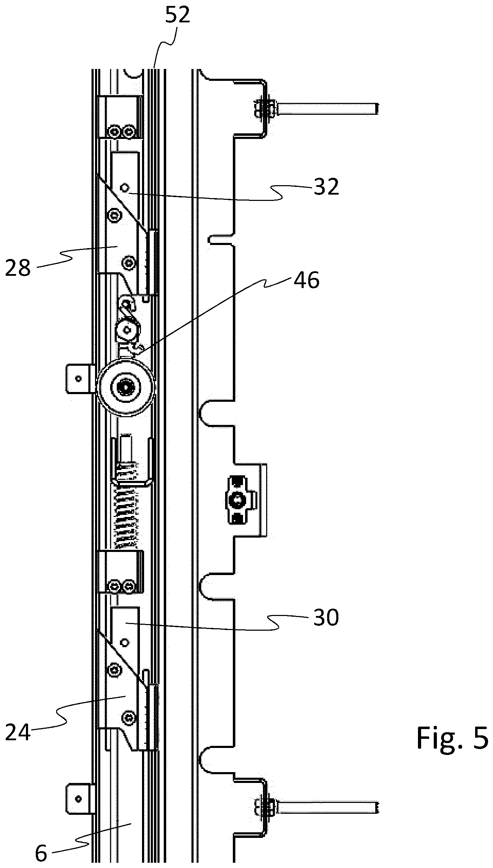

After the door 6 has reached the raised stop position, continued upward movement of the carrier arrangement 20 causes the first spring member 42 to become more compressed, thereby enabling the first slide surfaces 34, 38 of the slide members 30, 32 to slide along the respective mating second slide surfaces 36, 40 of the second and fourth carrier elements 24, 28. This causes the door 6 to move horizontally from the raised stop position (Fig. 4) to the raised closed position (Fig. 5) due to the continued upward movement of the carrier arrangement 20. In other words, when the door 6 is moved from the raised stop position (Fig. 4) to the raised closed position (Fig. 5), vertical movement of the door 6 is substantially prohibited, the first spring member 42 is compressed, and upwards pushing of the second slide surfaces 36, 40 against the first slide surfaces 34, 38 causes sliding by the first slide surfaces 34, 38 which moves the door 6 horizontally into the raised closed position illustrated in Fig. 5.

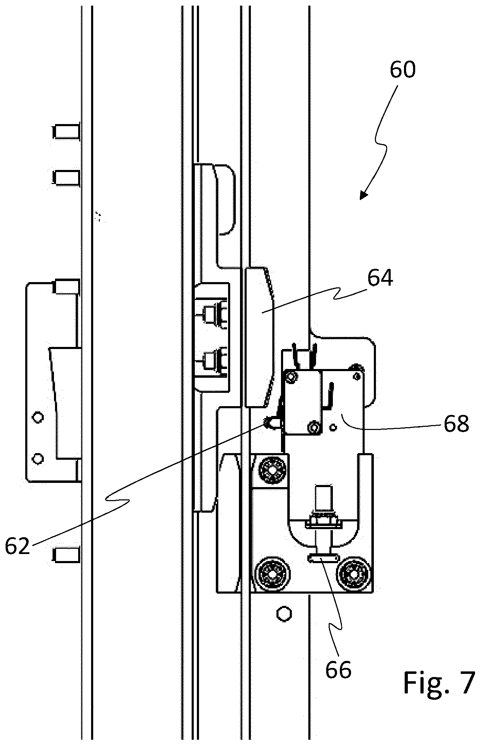

As can be seen in Fig. 5, the slide members 30, 32 have now moved along the second and fourth carrier elements 24, 28, respectively, in comparison to position of the slide members 30, 32 in Fig. 4. In the raised closed position of Fig. 5, the slide members 30, 32 are located at a relatively proximal position, i.e. proximal relative to the opening and the surrounding gasket 52. The medical washer/disinfector 1 may suitably be configured to allow an adjustable sealing pressure of the door 6 against the gasket 52. Thus, for the raised closed position illustrated in Fig. 5, the sealing pressure may suitably be selectable. This will now be exemplified in connection with Fig. 7.

Fig. 7 illustrates a detailed view of a part of the medical washer/disinfector, for example a lower part thereof, which is not visible in the other drawing figures. The medical washer/disinfector comprises an actuator mechanism 60 for enabling adjustment of the sealing pressure. The actuator mechanism 60 comprises a limit switch 62 and a shuttle 64. The shuttle 64 is configured to move in the opposite vertical direction relative to the carrier arrangement. Thus, when the carrier arrangement moves upwards, the shuttle 64 moves downwards, and when the carrier arrangement moves downwards, the shuttle 64 moves upwards. The shuttle 64 is configured to engage and activate the limit switch 62 during its downwards vertical movement when the door has moved from the raised stop position to the raised closed position. Activation of the limit switch 62 stops the carrier arrangement from moving further vertically upwards and thus stops the door from being pressed (by the second slide surfaces) further towards the opening and the surrounding gasket. The vertical position of the limit switch 62 is adjustable. In Fig. 7, this is illustrated by means of a turnable screw 66, which via a plate 68 moves the limit switch 62 up or down (depending on the direction in which you turn the screw 66). Thus, by setting a desired vertical position of the limit switch 62, you can decide how much more the carrier arrangement may continue to move upwards and thus continue to press the door against the gasket by (means of the slide surface interfaces) after the door has already reached the raised position. In this way the sealing pressure may be selected.

It has been noted that the selected sealing pressure may also affect the ability of the door to separate from the gasket when the door is to be opened. In order to facilitate proper separation of the door from the gasket and enabling the door to reach its laterally spaced apart raised stop position before being lowered into the lowered open position, the above mentioned second spring member has been provided.

Turning tow Fig. 6, the second spring member 46 is configured to accumulate elastic potential energy when the door is moved from the raised stop position to the raised closed position. When the door is in the raised closed position and the motor starts to move the carrier arrangement downwardly, the second spring member 46 releases the elastic potential energy to separate the door from the gasket. More specifically, when the door is moved from the raised stop position (Fig. 4) to the raised closed position (Fig. 5), the spring portion 50 pulls and turns the turnable wheel 48, as can be understood when comparing the different states of the second spring member 46 shown in Figs. 4 and 5. In this way, the energy is accumulated in the spring portion 50 and later released to assist in separating the door in the horizontal direction from the gasket 52.

## Claims

1. A medical washer/disinfector (1), comprising:
- a washing chamber (2) for receiving medical articles to be washed/disinfected,
- an opening (4) for accessing the interior of the washing chamber,
- a door (6) which is movable
∘ from a lowered open position, enabling medical articles to be inserted into or retrieved from the washing chamber through the opening,
∘ and then to a raised stop position, in which the door is located in front of the opening but positioned laterally spaced apart from the opening,
∘ and then to a raised closed position, in which the door is positioned against the opening and seals the opening,
wherein the door also passes through the raised stop position when transitioning from the raised closed position back to the lowered open position,
the door comprising or being fixedly connected to a first slide surface (34),
- a motor (14),
- a carrier arrangement (20) vertically movable by the motor, the carrier arrangement comprising a second slide surface (36) for enabling slidable contact with the first slide surface,
- a spring member (42) connected to the door at a connection area (44), the spring member being in contact with the carrier arrangement and extending vertically upwardly from the carrier arrangement to said connection area,
wherein when the door is in the lowered open position and the motor moves the carrier arrangement upwards, the door is pushed upwards by the spring member until the door reaches said raised stop position at which point further upwards movement of the door is prevented, wherein the pushing upwards by the spring member prevents sliding motion between the first and second slide surfaces during the movement of the door from the lowered open position to the raised stop position,
wherein, after the door has reached the raised stop position, continued upward movement of the carrier arrangement causes the spring member to become more compressed, thereby enabling the first slide surface to slide along the second slide surface, whereby the door moves horizontally from the raised stop position to the raised closed position due to the continued upward movement of the carrier arrangement.

2. The medical washer/disinfector (1) as claimed in claim 1, wherein at least one of the first and second slide surfaces (34, 36) is inclined such that it is at a higher vertical level distally to the washing chamber (2) and at a lower vertical level proximally to the washing chamber.

3. The medical washer/disinfector (1) as claimed in any one of claims 1-2, comprising at least one slide member (30, 32) fixedly connected to a lateral side of the door (6), wherein the slide member comprises said first slide surface (34), wherein said first slide surface is inclined such that it is at a higher vertical level distally to the washing chamber (2) and at a lower vertical level proximally to the washing chamber.

4. The medical washer/disinfector (1) as claimed in any one of claims 1-3, wherein the carrier arrangement (20) comprises a first carrier element (22) and a second carrier element (24), the first carrier element being located above and vertically spaced apart from the second carrier element, wherein the spring member (42) is in contact with the first carrier element and extending vertically upwardly from the first carrier element, wherein an upper side of the second carrier element presents said second slide surface (36).

5. The medical washer/disinfector (1) according to any one of claims 1-4, wherein when the door (6) is moved from the lowered open position to the raised stop position, the door substantially floats on the spring member (42) so that the second slide surface (36) substantially does not press against or slide along the first slide surface (34).

6. The medical washer/disinfector (1) according to claim 5, wherein when the door (6) is moved from the raised stop position to the raised closed position, vertical movement of the door is substantially prohibited, the spring member (42) is compressed, and upwards pushing of the second slide surface (36) against the first slide surface (34) causes sliding by the first slide surface which moves the door horizontally into the raised closed position.

7. The medical washer/disinfector (1) according to any one of claims 1-6, comprising a gasket (52) around the opening (4), wherein the door ((6) seals against the gasket when it is in its raised closed position.

8. The medical washer/disinfector (1) according to claim 7, wherein said spring member is a first spring member (42), the medical washer/disinfector further comprising a second spring member (46), wherein the second spring member is configured to accumulate elastic potential energy when the door (6) is moved from the raised stop position to the raised closed position, wherein when the door is in the raised closed position and the motor (14) starts to move the carrier arrangement (20) downwardly, the second spring member releases the elastic potential energy to separate the door from the gasket (52).

9. The medical washer/disinfector (1) according to claim 8, wherein said second spring member (46) comprises a turnable wheel (48) and a spring portion (50), wherein the turnable wheel is connected to the carrier arrangement (20) and wherein the spring portion interconnects the turnable wheel with the door (6), wherein when the door is moved from the raised stop position to the raised closed position, the spring portion pulls and turns the turnable wheel.

10. The medical washer/disinfector (1) according to any one of claims 1-9, wherein the motor (14) comprises a self-locking gearbox, thereby preventing the carrier arrangement (20) from moving uncontrollably downwards when the door (6) is in the raised closed position.

11. The medical washer/disinfector (1) according to any one of claims 1-10, further comprising:
- a limit switch (62), and
- a shuttle (64) configured to move in the opposite vertical direction relative to the carrier arrangement (20), and configured to engage and activate the limit switch during its downwards vertical movement when the door (6) has moved from the raised stop position to the raised closed position,
wherein activation of the limit switch stops the carrier arrangement from moving further vertically upwards and thus stops the door from moving further towards or pressing further against the opening (4),
wherein the vertical position of the limit switch is adjustable, thereby enabling adjustment of the sealing pressure of the door.

## Patentansprüche

1. Medizinisches Wasch-/Desinfektionsgerät (1), umfassend:
- eine Waschkammer (2) zum Aufnehmen medizinischer Gegenstände, die zu waschen/desinfizieren sind,
- eine Öffnung (4) zum Zugreifen auf das Innere der Waschkammer,
- eine Tür (6), die wie folgt bewegbar ist:
• aus einer abgesenkten offenen Position, wodurch es ermöglicht wird, dass medizinische Gegenstände durch die Öffnung in die Waschkammer eingeführt oder aus ihr herausgenommen werden,
• und darauffolgend in eine angehobene Anschlagsposition, in der sich die Tür vor der Öffnung befindet, jedoch seitlich von der Öffnung beabstandet positioniert ist,
• und darauffolgend in eine angehobene geschlossene Position, in der die Tür an der Öffnung positioniert ist und die Öffnung abdichtet,
wobei sich die Tür ebenfalls durch die angehobene Anschlagsposition bewegt, wenn sie von der angehobenen geschlossenen Position zurück in die abgesenkte offene Position übergeht,
wobei die Tür eine erste Gleitfläche (34) umfasst oder fest damit verbunden ist,
- einen Motor (14),
- eine Trägeranordnung (20), die durch den Motor vertikal bewegbar ist, wobei die Trägeranordnung eine zweite Gleitfläche (36) zum Ermöglichen gleitfähigen Kontakts mit der ersten Gleitfläche umfasst,
- ein Federglied (42), das mit der Tür an einem Verbindungsbereich (44) verbunden ist, wobei das Federglied mit der Trägeranordnung in Kontakt steht und sich vertikal von der Trägeranordnung zu dem Verbindungsbereich aufwärts erstreckt, wobei, wenn sich die Tür in der abgesenkten offenen Position befindet und der Motor die Trägeranordnung aufwärtsbewegt, die Tür durch das Federglied aufwärts geschoben wird, bis die Tür die angehobene Anschlagsposition erreicht, woraufhin eine weitere Aufwärtsbewegung der Tür verhindert wird, wobei das Aufwärtsschieben durch das Federglied eine Gleitbewegung zwischen der ersten und der zweiten Gleitfläche während der Bewegung der Tür von der abgesenkten offenen Position in die angehobene Anschlagsposition verhindert,
wobei, nachdem die Tür die angehobene Anschlagsposition erreicht hat, eine fortgesetzte Aufwärtsbewegung der Trägeranordnung veranlasst, dass das Federglied stärker zusammengedrückt wird, wodurch es ermöglicht wird, dass die erste Gleitfläche entlang der zweiten Gleitfläche gleitet, wobei sich die Tür aufgrund der fortgesetzten Aufwärtsbewegung der Trägeranordnung horizontal von der angehobenen Anschlagsposition in die angehobene geschlossene Position bewegt.

2. Medizinisches Wasch-/Desinfektionsgerät (1) nach Anspruch 1, wobei mindestens eine der ersten und der zweiten Gleitfläche (34, 36) derart geneigt ist, dass sie sich distal zu der Waschkammer (2) auf einer höheren vertikalen Ebene und proximal zu der Waschkammer auf einer niedrigeren vertikalen Ebene befindet.

3. Medizinisches Wasch-/Desinfektionsgerät (1) nach einem der Ansprüche 1-2, umfassend mindestens ein Gleitglied (30, 32), das fest mit einer seitlichen Seite der Tür (6) verbunden ist, wobei das Gleitglied die erste Gleitfläche (34) umfasst, wobei die erste Gleitfläche derart geneigt ist, dass sie sich distal zu der Waschkammer (2) auf einer höheren vertikalen Ebene und proximal zu der Waschkammer auf einer niedrigeren vertikalen Ebene befindet.

4. Medizinisches Wasch-/Desinfektionsgerät (1) nach einem der Ansprüche 1-3, wobei die Trägeranordnung (20) ein erstes Trägerelement (22) und ein zweites Trägerelement (24) umfasst, wobei sich das erste Trägerelement oberhalb des zweiten Trägerelements und vertikal von ihm beabstandet befindet, wobei das Federglied (42) mit dem ersten Trägerelement in Kontakt steht und sich von dem ersten Trägerelement vertikal aufwärts erstreckt, wobei eine obere Seite des zweiten Trägerelements die zweite Gleitfläche (36) darstellt.

5. Medizinisches Wasch-/Desinfektionsgerät (1) nach einem der Ansprüche 1-4, wobei, wenn die Tür (6) von der abgesenkten offenen Position in die angehobene Anschlagsposition bewegt wird, die Tür im Wesentlichen auf dem Federglied (42) schwebt, sodass die zweite Gleitfläche (36) im Wesentlichen nicht gegen die erste Gleitfläche (34) drückt oder an ihr entlang gleitet.

6. Medizinisches Wasch-/Desinfektionsgerät (1) nach Anspruch 5, wobei, wenn die Tür (6) von der angehobenen Anschlagsposition in die angehobene geschlossene Position bewegt wird, eine vertikale Bewegung der Tür im Wesentlichen verhindert wird, das Federglied (42) zusammengedrückt wird und Aufwärtsschieben der zweiten Gleitfläche (36) gegen die erste Gleitfläche (34) Gleiten durch die erste Gleitfläche veranlasst, das die Tür horizontal in die angehobene geschlossene Position bewegt.

7. Medizinisches Wasch-/Desinfektionsgerät (1) nach einem der Ansprüche 1-6, umfassend eine Dichtung (52) um die Öffnung (4), wobei die Tür (6) gegen die Dichtung abgedichtet wird, wenn sie sich in ihrer angehobenen geschlossenen Position befindet.

8. Medizinisches Wasch-/Desinfektionsgerät (1) nach Anspruch 7, wobei das Federglied ein erstes Federglied (42) ist, wobei das medizinische Wasch-/Desinfektionsgerät ferner ein zweites Federglied (46) umfasst, wobei das zweite Federglied dazu konfiguriert ist, elastische potenzielle Energie zu sammeln, wenn die Tür (6) von der angehobenen Anschlagsposition in die angehobene geschlossene Position bewegt wird, wobei, wenn sich die Tür in der angehobenen geschlossenen Position befindet und der Motor (14) anfängt, die Trägeranordnung (20) abwärtszubewegen, das zweite Federglied die elastische potenzielle Energie freigibt, um die Tür von der Dichtung (52) zu trennen.

9. Medizinisches Wasch-/Desinfektionsgerät (1) nach Anspruch 8, wobei das zweite Federglied (46) ein drehbares Rad (48) und einen Federabschnitt (50) umfasst, wobei das drehbare Rad mit der Trägeranordnung (20) verbunden ist und wobei der Federabschnitt das drehbare Rad mit der Tür (6) verbindet, wobei, wenn die Tür von der angehobenen Anschlagsposition in die angehobene geschlossene Position bewegt wird, der Federabschnitt das drehbare Rad zieht und dreht.

10. Medizinisches Wasch-/Desinfektionsgerät (1) nach einem der Ansprüche 1-9, wobei der Motor (14) ein selbstverriegelndes Getriebe umfasst, wodurch verhindert wird, dass sich die Trägeranordnung (20) unkontrolliert abwärtsbewegt, wenn sich die Tür (6) in der angehobenen geschlossenen Position befindet.

11. Medizinisches Wasch-/Desinfektionsgerät (1) nach einem der Ansprüche 1-10, ferner umfassend:
- einen Begrenzungsschalter (62) und
- ein Schiffchen (64), das dazu konfiguriert ist, sich in die entgegengesetzte vertikale Richtung in Bezug auf die Trägeranordnung (20) zu bewegen, und dazu konfiguriert ist, während seiner vertikalen Abwärtsbewegung den Begrenzungsschalter in Eingriff zu nehmen und zu betätigen, wenn sich die Tür (6) von der angehobenen Anschlagsposition in die angehobene geschlossene Position bewegt hat,
wobei das Betätigen des Begrenzungsschalters die Trägeranordnung daran hindert, sich weiter vertikal aufwärtszubewegen, und somit die Tür daran hindert, sich weiter auf die Öffnung (4) hinzubewegen oder weiter gegen sie zu drücken,
wobei die vertikale Position des Begrenzungsschalters einstellbar ist, wodurch Einstellen des Abdichtdrucks der Tür ermöglicht wird.

## Revendications

1. Agent de nettoyage/désinfection médical (1), comprenant :
- une chambre de lavage (2) destinée à recevoir des articles médicaux à laver/désinfecter,
- une ouverture (4) destinée à accéder à l'intérieur de la chambre de lavage,
- une porte (6) qui est mobile
• à partir d'une position ouverte abaissée, permettant aux articles médicaux d'être insérés dans ou récupérés de la chambre de lavage à travers l'ouverture,
• puis vers une position d'arrêt relevée, dans laquelle la porte est située devant l'ouverture mais positionnée latéralement à distance de l'ouverture,
• puis vers une position fermée relevée, dans laquelle la porte est positionnée contre l'ouverture et assure l'étanchéité de l'ouverture,
dans lequel la porte passe également par la position d'arrêt relevée lors du passage de la position fermée relevée à la position ouverte abaissée,
la porte comprenant ou étant reliée de manière fixe à une première surface coulissante (34),
- un moteur (14),
- un agencement de support (20) mobile verticalement par le moteur, l'agencement de support comprenant une seconde surface coulissante (36) pour permettre un contact coulissant avec la première surface coulissante,
- un élément à ressort (42) relié à la porte au niveau d'une zone de connexion (44), l'élément à ressort étant en contact avec l'agencement de support et s'étendant verticalement vers le haut depuis l'agencement de support jusqu'à ladite zone de connexion,
dans lequel, lorsque la porte est dans la position ouverte abaissée et que le moteur déplace l'agencement de support vers le haut, la porte est poussée vers le haut par l'élément à ressort jusqu'à ce que la porte atteigne ladite position d'arrêt relevée, moment auquel un mouvement supplémentaire vers le haut de la porte est empêché, dans lequel la poussée vers le haut par l'élément à ressort empêche le mouvement coulissant entre les première et seconde surfaces coulissantes pendant le mouvement de la porte de la position ouverte abaissée à la position d'arrêt relevée,
dans lequel, une fois que la porte a atteint la position d'arrêt relevée, un mouvement vers le haut continu de l'agencement de support amène l'élément à ressort à devenir davantage comprimé, permettant ainsi à la première surface coulissante de coulisser le long de la seconde surface coulissante, moyennant quoi la porte se déplace horizontalement de la position d'arrêt relevée à la position fermée relevée en raison du mouvement vers le haut continu de l'agencement de support.

2. Agent de nettoyage/désinfection médical (1) selon la revendication 1, dans lequel au moins l'une des première et seconde surfaces coulissantes (34, 36) est inclinée de sorte qu'elle se trouve à un niveau vertical plus élevé de manière distale par rapport à la chambre de lavage (2) et à un niveau vertical inférieur à proximité de la chambre de lavage.

3. Agent de nettoyage/désinfection médical (1) selon l'une quelconque des revendications 1 et 2, comprenant au moins un élément coulissant (30, 32) relié de manière fixe à un côté latéral de la porte (6), dans lequel l'élément coulissant comprend ladite première surface coulissante (34), dans lequel ladite première surface coulissante est inclinée de sorte qu'elle se trouve à un niveau vertical supérieur de manière distale par rapport à la chambre de lavage (2) et à un niveau vertical inférieur à proximité de la chambre de lavage.

4. Agent de nettoyage/désinfection médical (1) selon l'une quelconque des revendications 1 à 3, dans lequel l'agencement de support (20) comprend un premier élément de support (22) et un second élément de support (24), le premier élément de support étant situé au-dessus et espacé verticalement du second élément de support, dans lequel l'élément à ressort (42) est en contact avec le premier élément de support et s'étendant verticalement vers le haut à partir du premier élément de support, dans lequel un côté supérieur du second élément de support présente ladite seconde surface coulissante (36).

5. Agent de nettoyage/désinfection médical (1) selon l'une quelconque des revendications 1 à 4, dans lequel lorsque la porte (6) est déplacée de la position ouverte abaissée à la position d'arrêt relevée, la porte flotte sensiblement sur l'élément à ressort (42) de sorte que la seconde surface coulissante (36) n'appuie pratiquement pas contre la première surface coulissante (34) ni ne coulisse le long de celle-ci.

6. Agent de nettoyage/désinfection médical (1) selon la revendication 5, dans lequel lorsque la porte (6) est déplacée de la position d'arrêt relevée à la position fermée relevée, le mouvement vertical de la porte est sensiblement interdit, l'élément à ressort (42) est comprimé et la poussée vers le haut de la seconde surface coulissante (36) contre la première surface coulissante (34) provoque le coulissement de la première surface coulissante, ce qui déplace la porte horizontalement dans la position fermée relevée.

7. Agent de nettoyage/désinfection médical (1) selon l'une quelconque des revendications 1 à 6, comprenant un joint (52) autour de l'ouverture (4), dans lequel la porte (6) assure l'étanchéité contre le joint lorsqu'elle est dans sa position fermée relevée.

8. Agent de nettoyage/désinfection médical (1) selon la revendication 7, dans lequel ledit élément à ressort est un premier élément à ressort (42), l'agent de nettoyage/désinfection médical comprenant en outre un second élément à ressort (46), dans lequel le second élément à ressort est configuré pour accumuler l'énergie potentielle élastique lorsque la porte (6) est déplacée de la position d'arrêt relevée à la position fermée relevée, dans lequel lorsque la porte est dans la position fermée relevée et que le moteur (14) commence à déplacer l'agencement de support (20) vers le bas, le second élément à ressort libère l'énergie potentielle élastique pour séparer la porte du joint (52).

9. Agent de nettoyage/désinfection médical (1) selon la revendication 8, dans lequel ledit second élément à ressort (46) comprend une roue pouvant tourner (48) et une partie ressort (50), dans lequel la roue pouvant tourner est reliée à l'agencement de support (20) et dans lequel la partie ressort relie la roue pouvant tourner à la porte (6), dans lequel lorsque la porte est déplacée de la position d'arrêt relevée à la position fermée relevée, la partie ressort tire et fait tourner la roue pouvant tourner.

10. Agent de nettoyage/désinfection médical (1) selon l'une quelconque des revendications 1 à 9, dans lequel le moteur (14) comprend une boîte de vitesses à verrouillage automatique, empêchant ainsi l'agencement de support (20) de se déplacer de manière incontrôlable vers le bas lorsque la porte (6) est en position fermée relevée.

11. Agent de nettoyage/désinfection médical (1) selon l'une quelconque des revendications 1 à 10, comprenant en outre :
- un interrupteur de fin de course (62), et
- une navette (64) configurée pour se déplacer dans la direction verticale opposée par rapport à l'agencement de support (20), et configurée pour engager et activer l'interrupteur de fin de course pendant son mouvement vertical vers le bas lorsque la porte (6) s'est déplacée de la position d'arrêt relevée à la position fermée relevée,
dans lequel l'activation de l'interrupteur de fin de course empêche l'agencement de support de se déplacer davantage verticalement vers le haut et empêche ainsi la porte de se rapprocher davantage de l'ouverture (4) ou de s'appuyer davantage contre celle-ci,
dans lequel la position verticale de l'interrupteur de fin de course est réglable, permettant ainsi le réglage de la pression d'étanchéité de la porte.
